(19) **Europäisches Patentamt / European Patent Office / Office européen des brevets**

(11) **EP 2 949 315 B1**

(12) **EUROPEAN PATENT SPECIFICATION**

(45) Date of publication and mention
of the grant of the patent:
**19.05.2021 Bulletin 2021/20**

(21) Application number: **13859508.7**

(22) Date of filing: **16.07.2013**

(51) Int Cl.:
*A61K 9/02* *(2006.01)*     *A61F 13/20* *(2006.01)*
*A61F 13/32* *(2006.01)*     *A61M 31/00* *(2006.01)*
*A61K 9/00* *(2006.01)*

(86) International application number:
**PCT/CN2013/079450**

(87) International publication number:
**WO 2014/082456 (05.06.2014 Gazette 2014/23)**

(54) **EXPANSION CARRIER WITH ACTIVE COMPONENT**

EXPANSIONSTRÄGER MIT AKTIVER KOMPONENTE

VECTEUR DE DILATATION AVEC COMPOSANT ACTIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.11.2012 CN 201210499627**

(43) Date of publication of application:
**02.12.2015 Bulletin 2015/49**

(73) Proprietors:
• **Harbin OT Pharmaceutical Co. Ltd
Heilongjiang 150069 (CN)**
• **Qiu, Mingshi
Heilongjiang 150069 (CN)**

(72) Inventors:
• **QIU, Mingshi
Harbin
Heilongjiang 150069 (CN)**

• **QIU, Xueliang
Harbin
Heilongjiang 150069 (CN)**

(74) Representative: **Fabry, Bernd
IP2 Patentanwalts GmbH
Schlossstrasse 523
41238 Mönchengladbach (DE)**

(56) References cited:
**CN-A- 1 246 333       CN-A- 1 607 930
CN-A- 102 961 260     CN-A- 103 181 889
CN-U- 202 505 962     CN-Y- 2 247 541
US-A- 3 902 493       US-A- 4 983 163
US-A1- 2003 153 864   US-A1- 2007 141 118
US-A1- 2007 293 837**

## Description

### FIELD OF TECHNOLOGY

This invention belongs to the field of health care and pharmaceuticals and particularly relates to a type of expansion carrier containing active ingredients.

### TECHNOLOGICAL BACKGROUND

The new carrier material with specific swelling properties play an important role in the food and drug industries, in cosmetics, disinfectants, health care products, and medical devices. Various further profitable applications are possible. For instance, the common sterile tampon possesses a wide range of applications. It can be used as absorbent medical cotton, disposable sanitary products such as sanitary napkins during menstruation, and tampons combined with suppositories for internal use.

However, e.g. the cosmetics, disinfectant, and medical device industries differ greatly in product requirements. As a consequence, one carrier with specific physical and chemical properties can only be applied to some but not all areas. At present, the swelling properties of common absorbent cotton are irregular, and the simple and crude material contained constrains its wide usage in various fields.

US 2003/153864 A1 discloses a disposable device comprising an expandable tampon (6) made of at least one absorbent material and designed to collect and absorb the active liquid dispensed in the intracorporeal cavity, a source of liquid phase including a capsule enclosing a liquid charge at least the proximal part of which is at least partially surrounded by the expandable tampon, a covering for temporary protection at least partially covering the distal portion of said capsule, and extending longitudinally to an intermediate level of the tampon in such a way as to form in said tampon a proximal plug not covered by said covering, which is at least radially expandable independently from the remaining part of said tampon.

US 4 983 163 A discloses an applicator pad, wherein body may be a stretchable, knitted fabric of moisture absorbent textile into which the dried culture of L. acidophilus and/or L. bulgaricus may be incorporated.

US 2007/293837 A1 describes a delivery system including a reservoir formed of porous material; a flowable therapeutic formulation contained within the reservoir; and a covering formed of porous material enveloping the reservoir. According to US 2007/293837 A1 the reservoir material comprises an assembly of fibers having interfiber spaces defining pores within which the flowable therapeutic formulation is contained, wherein the reservoir comprises relatively coarse and stiff textile fibers.

US 2007/141118 A1 discloses a tampon adapted to deliver a therapeutic agent, the tampon comprising: a tampon body having a distal end; and a dosage form affixed to the distal end of the tampon body, the dosage form including a first active layer dispersible by a first mechanism, the active layer including the therapeutic agent, and an attachment layer dispersible by a second mechanism, wherein the first mechanism is different from the second mechanism.

US 3 902 493 A1 reports a medicament-bearing catamenial tampon, comprising: a resilient foam corpus having a modulus of compression which allows it to be reformed following insertion to conform to the irregularities of the vaginal wall; and a medicament-bearing hydrophobic non-woven overwrap which is permeable to vaginal fluids.

The following patents have improved the physical properties and applications of the absorbent cotton in different ways; for example Chinese patent CN02825892 comprises a special tampon for women, Chinese patent CN88105466 comprises a swelling cellulose sponge, Chinese patent CN200580022237 comprises a controllable intravaginal swelling device, Chinese patent CN200680014597 comprises a dual-mode absorption sanitary napkin, Chinese patent CN200880101828 comprises a tampon that can swell based on human anthropometric indices. The problems evolving from the "specific carrier with specific swelling-properties ", such as narrow application, simple and crude components, arbitrary swelling sequences, etc., are not completely resolved. Therefore, the invention of a new-type of expansion carrier with active ingredients demonstrates significant progress.

### DESCRIPTION OF THE INVENTION

The invention provides a new expansion carrier that can be used as disinfectants, drugs, cosmetics, and other products. According to the various active ingredients inside, the carrier can be manufactured as drugs, cosmetics and disinfectant products respectively.

According to the present application, an expansion carrier with active ingredients for intravaginal administration, characterized in that, the expansion carrier is made of one disappearing fiber, one natural fiber and one synthetic fiber in a ratio of 4-7:1 -3:1, the active ingredients are substances acting as therapeutics, health care, disinfectant, cosmetics, or cleansing products, and immerse into the carrier, dissolve or disperse into the surrounding compound of the carrier, wherein the weight ratio of the active ingredients and the expansion carrier is between 0.1:50 and 50:1, the swelling value of the expansion carrier obtained by melt time assay in the appendix of the Chinese Pharmacopoeia 2010 ed. is from 1-30 when it saturable absorbs water and on the surface of the carrier with active ingredients several ridges are deposed axially or radially or the expansion carrier is coated with a protective layer.

This expansion carrier with active ingredients of the present invention can swell in aqueous solution as per the preset swelling value. The administration of the expansion carrier is preferably intravaginal administration. However, the application range should not reduce the scope of protection for this invention.

According to the present invention the carried substances including the active ingredients can come in contact with the expansion carrier directly or indirectly.

As for cosmetics, active ingredients include but are not limited to liquor paraffin wax, lanolin, olive oil, carbopol, hyaluronic acid, white mineral oil, lanolin, Vaseline, vegetable oil, water-in-oil (W/O) cleansing cream and lotion, oil-in-water (O/W) cleansing cream and lotion, and surfactant.

Disinfection products include but are not limited to chlorhexidine diacetate, chitosan, formic acid, benzoic acid, salicylic acid, ethanol, surfactant, halogens, etc.

Drugs include but are not limited to various herbal and mineral medicinal extracts and ornidazole, terconazole, mandelic acid, bismuth subgallate, chlorhexidine diacetate, zedoary turmeric oil, metronidazole, chloramphenicol, sea-buckthorn seed oil, erythromycin, palmatine, flavonoids, furazolidone, policresulen, povidone-iodine, carboprost methylate, Kang Fu Ling, clindamycin phosphate, clotrimazole, matrine, bifonazole, morphine sulfate, sulfasalazine, Callicarpa nudiflora, indometacin and furazolidone, naproxen, diclofenac potassium, pyrantel pamoate, tegafur, tinidazole, ketoconazole, econazole nitrate, brufen, clenbuterol, levamisole hydrochloride, ofloxacin, nysfungin, tramadol hydrochloride, miconazole nitrate, nonoxinol, prostaglandin E2, meloxicam, mesalazine, ciclopirox olamine, compound carraghenates, dinoprostone, estriol, pegylated interferon $\alpha$2a in suppository form, butoconazole, fluconazole, fenticonazole, tioconazole, itraconazole, fungicidin, clindamycin hydrochloride, cyclomycin, doxycycline, lomefloxacin, norfloxacin, ciprofloxacin, azithromycin, and cefotaxime.

The 'preset swelling value' of the present invention is achieved by selection and proportion of the materials used for the expansion carrier. The preset swelling values facilitate sufficient contact of the active ingredients with the vaginal epithelium and mucosa, thus achieving selective contact. For example, the preferable materials with a preset swelling value and their proportion are as follows: the first expansion carrier should contain at least one disappearing fiber, one natural fiber and one synthetic fiber. The ratio of the three materials should be 4-7:1 -3:1, most preferably 5.5:2:1. Surprisingly, the expansion carrier can swell according to the preset swelling value in aqueous solution at pH 3.0 - 8.0.

In order to better fit the physiological structure of the vagina the swelling values can differ in different parts of the carrier. Some parts can also have the same swelling value but different swelling sequences.

On the surface of the expansion carrier with active ingredients several ridges are deposed axially or radially along the carrier. For the carrier with axial ridges, the swelling value of the head part and tail part are identical but the swelling sequence is different. Most preferably the tail part of the carrier swells first. For the carrier with radial ridges the whole carrier swells simultaneously. The parts of ridges possess a higher swelling value than the remaining parts. Most preferably the ridge of the tail part possesses the highest swelling value. By this way a "multiple leak proof effect" is established.

"Head part" is the part of the carrier which is inserted into the vagina first. 'Tail part' is the part of the carrier connected to the pull cord which serves for the removal of the carrier from the vagina.

When the swelling value is different, the regions with higher swelling values of the expansion carrier are preferably made of cellulose fibers, natural fibers, and synthetic fibers in the ratio of 7:1:1. The regions with lower swelling values are made of cellulose fibers, natural fibers, and synthetic fibers in the ratio of 4:3:1. The two regions can be arranged in various ways, e.g. in intervals or adjacently.

The experiments for measuring the 'swelling value of the carrier by saturable absorption' for different ridge directions were measured in the same way. The following example only refers to the measurement method for the radial swelling value. According to the melt time assay in the appendix of the Chinese Pharmacopoeia 2010 ed., three expansion carriers were left at room temperature for 1 hour. Afterwards, the tail part diameter (D1) of all three carriers was measured. The carriers were then placed into respective sheaths and fixed with hooks in a metal frame. The frame was placed vertically into a container filled with 4 L of water at 37.0 °C +/- 0.5 °C. The carriers were positioned 90 mm below the surface. A rotator was installed to flip the frame every 10 min. After 60 min, the tail part diameter (D2) was immediately measured when the carriers were removed from the container. The radial swelling value (S1) is defined as follows:

$$S_1 = D2/D1$$

$S_1$ is the mean value of the measured value for the 3 expansion carriers.

The swelling value of the carrier according to the present invention preferably ranges from 1 - 10, and a pull cord should be tied to its tail part. The cord can be made from sterile cotton, PVC, PE, nylon, cloth, or other materials.

The preferable size of the expansion carrier with active ingredients of the present invention is as follows: the diameter ranges between 3.0 - 15.0 mm, the weight ranges between 1.0 - 4.0 g, the carrier length lies between 10.0 - 60.0 mm, and the cord length is ≥ 50 mm. The most preferable parameters of the expansion carrier are as follows: the diameter ranges between 10.0 - 11.0 mm, the weight ranges between 2.10 - 2.40 g, the carrier length lies between 43.0 - 46.0 mm, and the cord length is ≥ 100 mm.

The expansion carrier is made of one disappearing fiber, one natural fiber and one synthetic fiber. Synthetic fibers include polyolefin, polyester, polyamide, rayon, high absorbent fiber, polyacrylonitrile fibers, viscose fiber and regenerated cellulose fiber. Natural fibers include wool, cotton, flax, hemp, jute, ramie, and wood pulp.

The present invention supplies another preferable technical solution, i.e. the expansion carrier with active ingredients is coated with a protective layer. The protective layer can restrict the swelling degree of the expansion carrier. For example, when a sterile tampon is used as the expansion carrier for loading the active ingredients, the p can be used as the protective layer.

Another preferable technical solution is, an internal expansion carrier can be placed into the expansion carrier with active ingredients of the present invention. The internal expansion carrier swells after the expansion carrier swells. Preferably there are two or more internal expansion carriers. If several internal expansion carriers are applied, one internal expansion carrier can be deposed into the other internal expansion carrier. These internal expansion carriers could also be placed into one expansion carrier or one internal expansion carrier side by side. According to the present invention, the weight ratio of the active ingredients and the expansion carrier is preferably between 0.1:50 and 50:1. When internal expansion carriers are deposed, the weight ratio of the active ingredient and the sum of carriers is 0.1:50 - 50:1.

The following examples are used to illustrate the application of the expansion carrier with active ingredients of the present invention.

First example is the use as a disinfectant. The disinfectant can be a bacteriostatic and vaginal care agent. The active ingredients include at least one kind of active substance for vaginal cleansing and at least one polymer excipients. The active substances include but are not limited to chlorhexidine acetate and chitosan. The polymer excipients include seaweed polysaccharide and liquid paraffin. The parts by weight of chlorhexidine acetate and chitosan are preferably both 100.

The second example is the use as cosmetics. The cosmetics are detergents for vaginal cleansing composed of an expansion carrier with at least one detergent. The cleaning products can further include a booster made from medicinal polyethylene or polypropylene material.

The booster is uniform in color. The surface is smooth, without distortions or visible scratches, free from blisters and fatty residues.

The preferable detergent is produced from the raw materials with the following parts by weight: liquor paraffin wax 30 - 60 %, lanolin 10 - 40 %, olive oil 10 - 40 %, carbopol 0.01 - 10 %, and hyaluronic acid 0.01 - 10 %.

The most preferable detergent has the following composition: liquor paraffin wax 44 %, lanolin 27 %, olive oil 28 %, carbopol 0.5 %, and hyaluronic acid 0.5 %.

The beneficial effects of the expansion carrier with active ingredients according to the present invention is its unique swelling performance according to the preset parameters, swelling under specific pH values, timed swelling, which make it as a new expansion carrier possible to be used for drugs, cosmetics, disinfectants and other products. A large number of experiments have shown that this carrier has a good performance in many different fields. As described in example 1, when it is used as disinfectant the growth inhibition rate of *Escherichia coli, Staphylococcus aureus* and *Candida albicans* is 50 % - 90 % after the solution is applied for 3 - 5 minutes.

## FIGURE DESCRIPTIONS

Figure 1: the structure drawing of the expansion carrier with active ingredients according to example 1

Figure 2: the structure drawing of the expansion carrier with active ingredients according to example 2

Figure 3: the structure drawing of the expansion carrier before swelling according to example 3

Figure 4: the structure drawing of the expansion carrier after swelling according to example 3

Figure 5: front view of the booster according to the present invention

Figure 6: separate parts of the booster according to the present invention

## EXAMPLES

Example 1: Bacteriostatic Agent

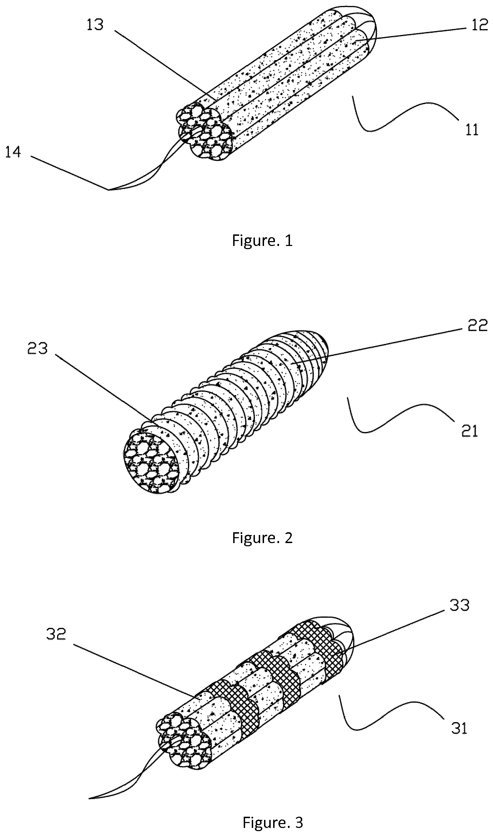

As shown in Figure 1, the main body (12) of the expansion carrier (11) with active ingredients of the present invention is made of cellulose fiber, jute and viscose fiber, with a proportion of 5.5:2:1. The cellulose fiber is provided by Shanghai Luoyang New Material Technology Co., Ltd.. several ridges (13) are deposed axially on the surface of the main bady (12). The tail part swells first but has the same swelling value as the head part. A cotton cord (14) is tied to the tail part of the expansion carrier.

The active ingredients loaded on the expansion carrier are chlorhexidine acetate and chitosan with a weight ratio of 100: 100, seaweed polysaccharide, and liquor paraffin wax.

When the device with bacteriostatic agent is placed into the vagina for 3 - 5 minutes, the growth inhibition rate of *Escherichia coli, Staphylococcus aureus* and *Candida albicans* is 50 % - 90 %.

Example 2: Cleaning Products

As shown in Figure 2, the expansion carrier with active ingredients is labeled as 21. Several ridges (23) are deposed axially on the surface of the main body (22). The ridges are the high swelling part, while the other sections are the low swelling part. The high swelling part of the expansion carrier is composed of cellulose fiber, cotton, and viscose fiber in a ratio of 7:1:1. The low swelling part is composed of cellulose fiber, cotton and viscose fiber in a ratio of 4:3:1. The cellulose fiber is provided by Shanghai Luoyang New Material Technology Co., Ltd. The tail part and head part swell simultaneously, but with different swelling values.

The active ingredients loaded in the expansion carrier of this example include: liquor paraffin wax 44 %, lanolin 27 %, olive oil 28 %, carbopol 0.5 %, and hyaluronic acid 0.5 %.

Example 3: Limited Expansion carrier

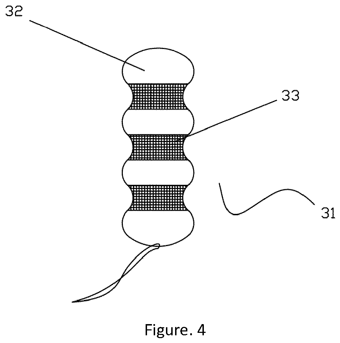

Figure 3 shows an expansion carrier (31) with active ingredients before swelling, including a sterile tampon (32) wrapped in three non-woven parts (33). In combination with the state after swelling showed in Figure 4, it can be proved that the swelling degree can be restricted by the non-woven fabric.

Example 4

This example is similar to example 1, with the exception that the used material is sterile tampon. The diameter of the most preferable expansion carrier is 10.0 mm; the weight is 2.20 g, the carrier length is 43.0 mm, and the cord length is 150 mm.

Example 5

This example is similar to example 2, with the exception that the used material is a sterile tampon. The diameter of the most preferable expansion carrier is 11.0 mm; the weight is 2.40 g, the carrier length is 46.0 mm, and the cord length is 200 mm.

Example 6

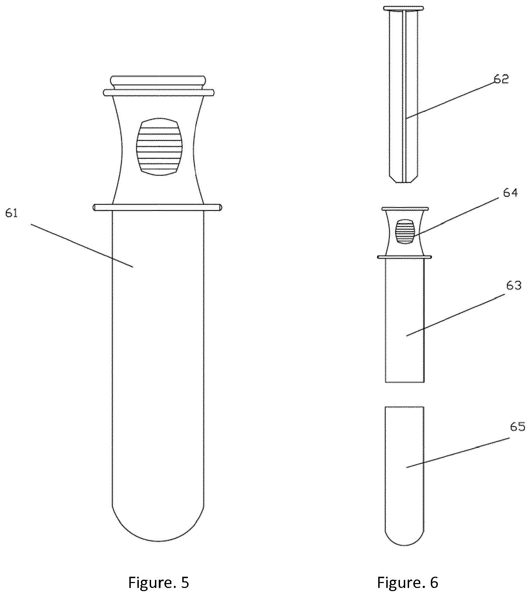

As shown in Figure 5 and 6, the booster includes a booster main body (63), and a plunger (62) which can slide through the main body, the booster main body (63) is hollow. A handle (64) is deposed on the upper part of the main body (63). The lower part is connected to a sheath (65).

**Claims**

1. An expansion carrier with active ingredients for intravaginal administration, **characterized in that**, the expansion carrier is made of one disappearing fiber, one natural fiber and one synthetic fiber in a ratio of 4-7:1 -3:1, the active ingredients are substances acting as therapeutics, health care, disinfectant, cosmetics, or cleansing products, and immerse into the carrier, dissolve or disperse into the surrounding compound of the carrier
wherein the weight ratio of the active ingredients and the expansion carrier is between 0.1:50 and 50:1, the swelling value of the expansion carrier obtained by melt time assay in the appendix of the Chinese Pharmacopoeia 2010 ed . is from 1-30 when it saturable absorbs water and on the surface of the carrier with active ingredients several ridges (13) are deposed axially or radially or the expansion carrier is coated with a protective layer.

2. The expansion carrier with active ingredients according to claim 1, wherein a pull cord is attached to the tail part of the expansion carrier.

3. The expansion carrier with active ingredient according to claim 2, wherein the diameter of the expansion carrier is 3.0 - 15.0 mm, the weight is 1.0 - 4.0 g, the carrier length is 10.0 - 60.0 mm, and the cord length is $\geq$ 50 mm, preferably the diameter is 10.0 - 11.0 mm, the weight is 2.10 - 2.40 g, the carrier length is 43.0 - 46.0 mm, and the cord length is $\geq$ 100 mm.

4. The expansion carrier with active ingredient according to claim 1, wherein the synthetic fibers include polyolefin, polyester, polyamide, rayon, high absorbent fiber, polyacrylonitrile, viscose fiber and regenerated cellulose fiber; the natural fibers include wool, cotton, flax, hemp, jute, ramie, and wood pulp.

5. The expansion carrier with active ingredient according to claim 4, wherein the expansion carrier can expand according to a preset swelling value in aqueous solution at pH 3.0 - 8.0.

6. The expansion carrier with active ingredient according to claim 1, wherein the expansion carrier is made of one disappearing fiber, one natural fiber and one synthetic fiber in a ratio of 5.5:2:1.

**Patentansprüche**

1. Expansionsträger mit Wirkstoffen zur intravaginalen Verabreichung, **dadurch gekennzeichnet, dass** der Expansionsträger aus einer sich auflösenden Faser, einer natürlichen Faser und einer synthetischen Faser in einem Verhältnis von 4-7:1-3:1 hergestellt ist, die Wirkstoffe Substanzen sind, die als Therapeutika, Gesundheitspflege, Desinfektionsmittel, Kosmetika oder Reinigungsprodukte wirken und in den Träger eintauchen, sich auflösen oder in die umgebende Verbindung des Trägers dispergieren,

wobei das Gewichtsverhältnis der Wirkstoffe und des Expansionsträgers zwischen 0,1:50 und 50:1 liegt, der Quellwert des Expansionsträgers, der durch den Schmelzzeittest im Anhang des Chinesischen Arzneibuchs 2010 Ed. beschrieben wird, 1 bis 30 beträgt, wenn er bis zur Sättigung Wasser absorbiert, und

auf der Oberfläche des Trägers mit Wirkstoffen mehrere Rippen (13) axial oder radial angebracht sind oder der Expansionsträger mit einer Schutzschicht überzogen ist.

**2.** Expansionsträger mit Wirkstoffen nach Anspruch 1, **dadurch gekennzeichnet, dass** am Endteil des Expansionsträgers eine Zugschnur angebracht ist.

**3.** Expansionsträger mit Wirkstoffen nach Anspruch 2, wobei der Durchmesser des Expansionsträgers 3,0 bis 15,0 mm, das Gewicht 1,0 bis 4,0 g, die Trägerlänge 10,0 bis 60,0 mm und die Schnurlänge 50 mm und

vorzugsweise der Durchmesser 10,0 bis 11,0 mm, das Gewicht 2,10 bis 2,40 g, die Trägerlänge 43,0 bis 46,0 mm und die Schnurlänge 100 mm beträgt.

**4.** Expansionsträger mit Wirkstoffen nach Anspruch 1, wobei die synthetischen Fasern Polyolefin, Polyester, Polyamid, Rayon, hochsaugfähige Faser, Polyacrylnitril, Viskosefaser sowie regenerierte Zellulosefaser und die natürlichen Fasern Wolle, Baumwolle, Flachs, Hanf, Jute, Ramie und Holzpulpe umfassen.

**5.** Expansionsträger mit Wirkstoffen nach Anspruch 4, wobei das Expansionsmittel in wässriger Lösung bei pH 3,0 - 8,0 entsprechend einem vorgegebenen Quellwert expandieren kann.

**6.** Expansionsträger mit Wirkstoffen nach Anspruch 1, wobei der Expansionsträger aus einer sich auflösenden Faser, einer Naturfaser und einer synthetischen Faser im Verhältnis 5,5:2:1 hergestellt ist.


**Revendications**

**1.** Un support d'expansion contenant des ingrédients actifs pour l'administration intravaginale, **caractérisé en ce que** le support d'expansion est fait d'une fibre dissolvante, d'une fibre naturelle et d'une fibre synthétique dans un rapport de 4-7:1-3:1, les ingrédients actifs sont des substances agissant comme des produits thérapeutiques, de soins de santé, désinfectants, cosmétiques ou de nettoyage et sont immergés dans le support, se dissolvent ou se dispersent dans le composé environnant du support,

dans lequel le rapport pondéral des ingrédients actifs et du support d'expansion est compris entre 0,1:50 et 50:1, la valeur de gonflement du support d'expansion décrite par le test de temps de fusion dans l'annexe de la Pharmacopée chinoise 2010 ed. est de 1 à 30 lorsqu'il absorbe de l'eau à saturation, et une pluralité de nervures (13) sont prévues axialement ou radialement sur la surface du support avec des ingrédients actifs, ou le support d'expansion est revêtu d'une couche protectrice.

**2.** Le support d'expansion avec des ingrédients actifs selon la revendication 1, **caractérisé en ce qu'**une corde de traction est fixée à la partie d'extrémité du support d'expansion.

**3.** Le support d'expansion avec des ingrédients actifs selon la revendication 2, dans lequel le diamètre du support d'expansion est de 3,0 à 15,0 mm, le poids est de 1,0 à 4,0 g, la longueur du support est de 10,0 à 60,0 mm, et la longueur du cordon est de 50 mm, et de préférence, le diamètre est de 10,0 à 11,0 mm, le poids est de 2,10 à 2,40 g, la longueur du support est de 43,0 à 46,0 mm et la longueur du cordon est de 100 mm.

**4.** Le support d'expansion avec des ingrédients actifs selon la revendication 1, dans lesquels les fibres synthétiques comprennent la polyoléfine, le polyester, le polyamide, la rayonne, la fibre hautement absorbante, le polyacrylonitrile, la fibre de viscose et la fibre de cellulose régénérée, et les fibres naturelles comprennent la laine, le coton, le lin, le chanvre, le jute, la ramie et la pulpe de bois.

**5.** Le support d'expansion avec des ingrédients actifs selon la revendication 4, dans lesquels l'agent d'expansion peut se dilater en solution aqueuse à pH 3,0 - 8,0 selon une valeur de gonflement prédéterminée.

**6.** Le support d'expansion avec des ingrédients actifs selon la revendication 1, dans lequel le support d'expansion est constitué d'une fibre dissolvante, d'une fibre naturelle et d'une fibre synthétique dans le rapport 5,5:2:1.

Figure. 1

Figure. 2

Figure. 3

32

33

31

Figure. 4

61

Figure. 5

62

64

63

65

Figure. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2003153864 A1
- US 4983163 A
- US 2007293837 A1
- US 2007141118 A1
- US 3902493 A1
- CN 02825892
- CN 88105466
- CN 200580022237
- CN 200680014597
- CN 200880101828

### Non-patent literature cited in the description

- Chinese Pharmacopoeia. 2010